# EUROPÄISCHE PATENTSCHRIFT

(11) **EP 1 466 903 B1**
(45) Veröffentlichungstag und Bekanntmachung des Hinweises auf die Patenterteilung: **02.06.2010**
(21) Anmeldenummer: 04002385.5
(22) Anmeldetag: 04.02.2004
(51) Int. Cl.: C07D 207/412

(54) **Korrosions- und Gashydratinhibitoren mit verbesserter Wasserlöslichkeit und erhöhter biologischer Abbaubarkeit**
Inhibitors of corrosion and of gas hydrates with an improved solubility in water and enhanced biodegradability
Inhibiteurs de corrosion et d'hydrates de gaz ayant une meilleure solubilité dans l'eau et une biodégradabilité ameliorée

(30) Priorität: 24.02.2003 DE 10307725
(43) Veröffentlichungstag der Anmeldung: 13.10.2004
(73) Patentinhaber: Clariant Produkte (Deutschland) GmbH, 65929 Frankfurt am Main (DE)
(72) Erfinder: Dahlmann, Uwe, Dr., 69126 Heidelberg (DE); Feustel, Michael, Dr., 55278 Köngernheim (DE)

(56) Entgegenhaltungen:
- EP-A- 0 154 740
- EP-A- 0 446 616
- EP-A- 0 473 229
- EP-A- 0 584 711
- WO-A-96/34177
- WO-A-98/03615
- DE-A- 2 559 189
- US-A- 3 017 416
- US-A- 3 113 026
- US-A- 4 171 959
- US-A- 5 254 138
- DATABASE CHEMCATS [ONLINE] CHEMICAL ABSTRACTS SERVICE, COLUMBUS, OHIO, US; zugänglich durch STN (chemcats 2002:1945168); XP002285322 & "Interchim Intermediates" 9. Juli 2002 (2002-07-09), INTERCHIM , MONTLUCON, CEDEX, 03103, FRANCE
- PATENT ABSTRACTS OF JAPAN Bd. 0176, Nr. 23 (M-1511), 17. November 1993 (1993-11-17) & JP 5 193073 A (TORAY IND INC), 3. August 1993 (1993-08-03)
- S L SHAPIRO, I M ROSE, L FREEDMAN: "Aminoalkylamides and Oxazolidinediones" JOURNAL OF THE AMERICAN CHEMICAL SOCIETY, Bd. 81, Nr. 12, 1959, Seiten 3083-3088, XP002285705
- L M WERBEL, E F ELSLAGER, P J ISLIP, M D CLOSIER: "Antischistosomal Effects of 5-(2,4,5-Trichlorophenyl)hydantoin and Related Compounds" JOURNAL OF MEDICINAL CHEMISTRY, Bd. 20, Nr. 12, 1977, Seiten 1569-1572, XP002285706
- C LION, L DA CONCEICAO, M HEDAYATULLAH: "Synthèse de nouveaux initiateurs. Génération in situ de composés peroxygènes par action du percarbonate de sodium. application à la destruction de toxiques organophosphores et/ou soufres" PHOSPORUS, SULFUR AND SILICON, Bd. 161, 2000, Seiten 97-113, XP009032524
- L CEKUOLIENE: "Aminomethylation of (+-)-1,2-bis(3,5-dioxopiperazin-1-yl)propa ne" LIETUVOS TSR MOKSLU AKADEMIJOS DARBAI, SERIJA B, CHEMIJA, TECHNIKA, FIZINE GEOGRAFIJA, Bd. 5, 1986, Seiten 41-45, XP009032530
- P MESSINGER, J GOMPERTZ: "Notiz zur Synthese von alpha-Amino- und alpha-Amidosulfonen. 5. Mitteilung über Sulfone als chemische Transportformen germicid wirkender Stoffe" ARCHIV DER PHARMAZIE, Bd. 307, Nr. 8, 1974, Seiten 653-655, XP009032537

## Beschreibung

Die vorliegende Erfindung betrifft ein Additiv und ein Verfahren zur Korrosionsinhibierung und Gashydratinhibierung an und in Einrichtungen zur Förderung und Transport von Kohlenwasserstoffen in der Erdölförderung und -verarbeitung.

In technischen Prozessen, bei denen Metalle mit Wasser oder auch mit ÖI-Wasser-Zweiphasensystemen in Kontakt kommen, besteht die Gefahr der Korrosion. Die ist besonders ausgeprägt, wenn die wässrige Phase wie bei Erdölgewinnungs- und Verarbeitungsprozessen stark salzhaltig oder durch gelöste Sauergase, wie Kohlendioxid bzw. Schwefelwasserstoff, azid ist. Daher ist die Ausbeutung einer Lagerstätte und die Verarbeitung von Erdöl ohne spezielle Additive zum Schutz der eingesetzten Ausrüstungen nicht möglich.

Geeignete Korrosionsschutzmittel für die Erdölförderung und -verarbeitung sind zwar schon seit langem bekannt, jedoch aus Gründen des Umweltschutzes für Offshore-Anwendungen zukünftig inakzeptabel.

Als typische Korrosionsinhibitoren des Standes der Technik besitzen Amide, Amidoamine bzw. Imidazoline von Fettsäuren und Polyaminen eine äußerst gute Öllöslichkeit und sind somit in der korrosiven Wasserphase aufgrund schlechter Verteilungsgleichgewichte (Partitioning) nur in geringer Konzentration vorhanden. Demgemäss müssen diese Produkte trotz ihrer schlechten biologischen Abbaubarkeit in hoher Dosierung eingesetzt werden.

DE-A-199 30 683 beschreibt entsprechende Amidoamine/Imidazoline, die durch Umsetzung von Alkylpolyglykolethercarbonsäuren mit Polyaminen erhalten werden, und die aufgrund eines besseren Partitioning in geringeren Konzentrationen eingesetzt werden können.

Quartäre Alkylammoniumverbindungen (Quats) stellen alternative Korrosionsschutzmittel des Standes der Technik dar, die neben den korrosionsinhibierenden auch biostatische Eigenschaften besitzen können. Trotz einer verbesserten Wasserlöslichkeit zeigen die Quats, zum Beispiel im Vergleich zu den Imidazolinen, eine deutlich reduzierte Filmpersistenz und führen daher ebenfalls nur in höherer Dosierung zu einem effektiven Korrosionsschutz. Die starke Algentoxizität und die mäßige biologische Abbaubarkeit beschränken den Einsatz von Quats immer mehr auf ökologisch unsensible Anwendungsgebiete, z.B. Onshore.

EP-B-0 946 788 beschreibt ein Verfahren zum Schutz von Metalloberflächen gegenüber Korrosion unter Verwendungen von Esterquats, von denen eine gute biologische Abbaubarkeit und eine geringe Aquatoxizität offenbart wird.

EP-A-0 320 769 offenbart gegebenenfalls quaternierte Fettsäureester von oxalkylierten Alkylamino-alkylenaminen und deren Verwendung als Korrosionsinhibitor.

EP-B-0 212 265 beschreibt quartäre Polykondensate aus alkoxylierten Alkylaminen und Dicarbonsäuren und deren Verwendung als Korrosionsinhibitor und Spalter in Rohölen.

EP-B-0 446 616 beschreibt Ampholyte und Betaine auf Basis Fettsäure-Amidoalkylaminen, die unter den gegebenen Testbedingungen einen sehr guten Korrosionsschutz und eine wesentlich reduzierte Algentoxizität aufweisen.

EP-B-0 584 711 offenbart Ester, Amide bzw. Imide von Alkenylbernsteinsäuren mit Alkoxyalkylaminen und deren Metall- bzw. Ammoniumsalze als Emulgatoren und Korrosionsinhibitoren für Metallbearbeitungshilfsmittel. Die Verwendung von Alkenylbernsteinsäure-ester- bzw. amidoaminquats oder entsprechenden Betainen ist nicht beschrieben.

Gashydrate sind kristalline Einschlussverbindungen von Gasmolekülen in Wasser, die sich unter bestimmten Temperatur- und Druckverhältnissen (niedrige Temperatur und hoher Druck) bilden. Hierbei bilden die Wassermoleküle Käfigstrukturen um die entsprechenden Gasmoleküle aus. Das aus den Wassermolekülen gebildete Gittergerüst ist thermodynamisch instabil und wird erst durch die Einbindung von Gastmolekülen stabilisiert. Diese eisähnlichen Verbindungen können in Abhängigkeit von Druck und Gaszusammensetzung auch über den Gefrierpunkt von Wasser (bis über 25°C) hinaus existieren.

In der Erdöl- und Erdgasindustrie sind insbesondere die Gashydrate von großer Bedeutung, die sich aus Wasser und den Erdgasbestandteilen Methan, Ethan, Propan, Isobutan, n-Butan, Stickstoff, Kohlendioxid und Schwefelwasserstoff bilden. Insbesondere in der heutigen Erdgasförderung stellt die Existenz dieser Gashydrate ein großes Problem dar, besonders dann, wenn Nassgas oder Mehrphasengemische aus Wasser, Gas und Alkangemischen unter hohem Druck niedrigen Temperaturen ausgesetzt werden. Hier führt die Bildung der Gashydrate aufgrund ihrer Unlöslichkeit und kristallinen Struktur zur Blockierung verschiedenster Fördereinrichtungen, wie Pipelines, Ventilen oder Produktionseinrichtungen, in denen über längere Strecken bei niedrigeren Temperaturen Nassgas oder Mehrphasengemische transportiert werden, wie dies speziell in kälteren Regionen der Erde oder auf dem Meeresboden vorkommt.

Außerdem kann die Gashydratbildung auch beim Bohrvorgang zur Erschließung neuer Gas- oder Erdöllagerstätten bei entsprechenden Druck- und Temperaturverhältnissen zu Problemen führen, indem sich in den Bohrspülflüssigkeiten Gashydrate bilden.

Um solche Probleme zu vermeiden, kann die Gashydratbildung in Gaspipelines, beim Transport von Mehrphasengemischen oder in Bohrspülflüssigkeiten durch Einsatz von größeren Mengen (mehr als 10 Gew.-% bezüglich des Gewichts der Wasserphase) niederen Alkoholen, wie Methanol, Glykol, oder Diethylenglykol unterdrückt werden. Der Zusatz dieser Additive bewirkt, dass die thermodynamische Grenze der Gashydratbildung zu niedrigeren Temperaturen und höheren Drücken hin verlagert wird (thermodynamische Inhibierung). Durch den Zusatz dieser thermodynamischen Inhibitoren werden allerdings größere Sicherheitsprobleme (Flammpunkt und Toxizität der Alkohole), logistische Probleme (große Lagertanks, Recycling dieser Lösungsmittel) und dementsprechend hohe Kosten, speziell in der Offshore-Förderung, verursacht.

Heute versucht man deshalb, thermodynamische Inhibitoren zu ersetzen, indem man bei Temperatur- und Druckbereichen, in denen sich Gashydrate bilden können, Additive in Mengen < 2 % zusetzt, die die Gashydratbildung entweder zeitlich hinauszögern (kinetische Inhibitoren) oder die Gashydratagglomerate klein und damit pumpbar halten, so dass diese durch die Pipeline transportiert werden können (sog. Agglomerat-Inhibitoren oder Anti-Agglomerates). Die dabei eingesetzten Inhibitoren behindern entweder die Keimbildung und/oder das Wachstum der Gashydratpartikel, oder modifizieren das Hydratwachstum derart, dass kleinere Hydratpartikel resultieren.

Als Gashydratinhibitoren wurden in der Patentliteratur neben den bekannten thermodynamischen Inhibitoren eine Vielzahl monomerer als auch polymerer Substanzklassen beschrieben, die kinetische Inhibitoren oder Agglomeratinhibitoren darstellen. Von besonderer Bedeutung sind hierbei Polymere mit Kohlenstoff Backbone, die in den Seitengruppen sowohl cyclische (Pyrrolidon- oder Caprolactamreste) als auch acyclische Amidstrukturen enthalten.

EP-B-0 736 130 offenbart ein Verfahren zur Inhibierung von Gashydraten, welches die Zuführung einer Substanz der Formel 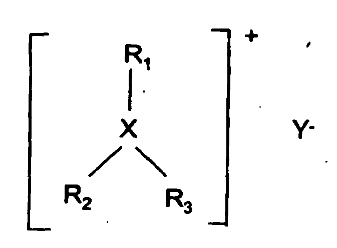 mit X = S, N - R₄ oder P - R₄, R₁, R₂ und R₃ = Alkyl mit mindestens 4 Kohlenstoffatomen, R₄ = H oder ein organischer Rest, und Y = Anion erfordert.

Umfasst werden also Verbindungen der Formel 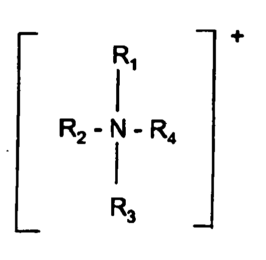 worin R₄ ein beliebiger Rest sein kann, die Reste R₁ bis R₃ aber Alkylreste mit mindestens 4 Kohlenstoffatomen darstellen müssen.
EP-B-0 824 631 offenbart ein Verfahren zur Inhibierung von Gashydraten, welches die Zuführung einer Substanz der Formel 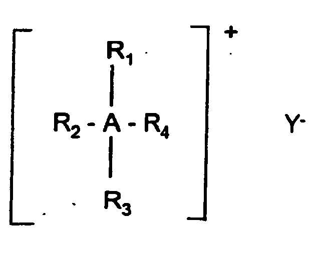 mit R₁, R₂ = gerade/verzweigte Alkylreste mit 4 oder 5 Kohlenstoffatomen, R₃, R₄ = organische Reste mit mindestens 8 Kohlenstoffatomen und A = Stickstoff oder Phosphor erfordert. Y⁻ ist ein Anion. Es müssen zwei der Reste R₁ bis R₄ gerade oder verzweigte Alkylreste mit 4 oder 5 Kohlenstoffatomen sein.

US-5 648 575 offenbart ein Verfahren zur Inhibierung von Gashydraten. Das Verfahren umfasst die Verwendung einer Verbindung der Formel 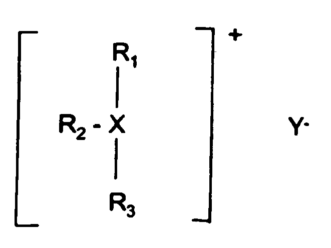 worin R¹, R² gerade oder verzweigte Alkylgruppen mit mindestens 4 Kohlenstoffatomen, R³ ein organischer Rest mit mindestens 4 Atomen, X Schwefel, NR⁴ oder PR⁴, R⁴ Wasserstoff oder ein organischer Rest, und Y ein Anion sind. Das Dokument offenbart nur solche Verbindungen, die mindestens zwei Alkylreste mit mindestens 4 Kohlenstoffatomen aufweisen.

US-6 025 302 offenbart Polyetheramin-ammoniumverbindungen als Gashydratinhibitoren, deren Ammoniumstickstoffatom neben der Polyetheraminkette 3 Alkylsubstituenten trägt.

WO-99/13197 offenbart Ammoniumverbindungen als Gashydratinhibitoren, die wenigstens eine mit Alkylcarbonsäuren veresterte Alkoxygruppe besitzen. Die Vorteile einer Verwendung von Alkenylbersteinsäurederivaten ist nicht offenbart.

WO-01/09082 offenbart ein Verfahren zur Herstellung von quartären Aminen, die jedoch keine Alkoxygruppen tragen, und deren Verwendung als Gashydratinhibitoren.

WO-00/078 706 offenbart quartäre Ammoniumverbindungen als Gashydratinhibitoren, die jedoch keine Carbonylreste tragen.

EP-B-914407 offenbart die Verwendung von trisubstituierten Aminoxiden als Gashydratinhibitoren.

US-5 254 138 offenbart Detergent-Additive für Dieselkraftstoff, die Succinimid-Polyaminderivate umfassen.

Aufgabe der vorliegenden Erfindung war es, neue Korrosionsinhibitoren zu finden, die bei konstant gutem oder verbessertem Korrosionsschutz neben einer optimierten Wasserlöslichkeit, einer schnelleren Filmbildung und somit verbesserten Filmpersistenz auch eine verbesserte biologische Abbaubarkeit im Vergleich zu den Korrosionsinhibitoren des Standes der Technik bieten.

Aufgabe der vorliegenden Erfindung war es ferner, verbesserte Additive zu finden, die sowohl die Bildung von Gashydraten verlangsamen (kinetische Inhibitoren) als auch Gashydratagglomerate klein und pumpbar halten (Anti-Agglomerates), um so ein breites Anwendungsspektrum mit hohem Wirkpotential zu gewährleisten. Des weiteren sollten die derzeit verwendeten thermodynamischen Inhibitoren (Methanol und Glykole), die beträchtliche Sicherheitsprobleme und Logistikprobleme verursachen, ersetzt werden können.

Gashydratinhibitoren des Standes der Technik werden gewöhnlich mit Korrosionsinhibitoren co-additiviert, um Korrosion der Transport- und Fördereinrichtungen vorzubeugen. Aufgrund der häufig nicht unmittelbar gegebenen Verträglichkeit von Gashydratinhibitor und Korrosionsschutzmittel bei der Formulierung ergibt sich daraus für den Anwender ein zusätzlichen Aufwand. Ein wesentlicher Vorteil gegenüber dem Stand der Technik bestände, wenn eine Co-Additivierung mit Korrosionsinhibitoren nicht mehr zwingend erforderlich ist.

Es wurde nun überraschenderweise gefunden, dass quartäre Alkylaminoalkyl/alkoxy-imide von Dicarbonsäuren eine ausgezeichnete Wirkung als Korrosionsinhibitoren und Gashydratinhibitoren aufweisen, sowie eine verbesserte Filmpersistenz und gute biologische Abbaubarkeit zeigen.

Gegenstand der Erfindung sind Verbindungen der Formel (1) 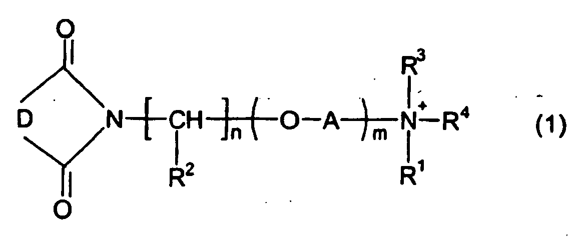 worin
- R¹: C₁- bis C₂₂-Alkyl, C₂- bis C₂₂-Alkenyl, C₆- bis C₃₀-Aryl- oder C₇- bis C₃₀-Alkylaryl, -CHR⁵-COO⁻ oder -O⁻,
- R²: Wasserstoff, -CH₃ oder -OH,
- R³, R⁴: unabhängig voneinander C₁- bis C₂₂-Alkyl, C₂- bis C₂₂-Alkenyl, C₆- bis C₃₀-Aryl- oder C₇- bis C₃₀-Alkylaryl,
- R⁵: Wasserstoff, C₁- bis C₂₂-Alkyl oder C₂- bis C₂₂-Alkenyl,
- A: eine C₂- bis C₄-Alkylengruppe,
- D: eine C₂- bis Alkylengruppe, die an jeder Position einen Substituenten R⁶ tragen kann,
- R⁶: einen beliebigen organischen Rest, der 1 bis 300 C-Atome enthält, und der Heteroatome enthalten kann,
- m: eine Zahl von 1 bis 30,
- n: eine Zahl von 1 bis 18 bedeuten,
wobei, wenn R¹ für C₁- bis C₂₂-Alkyl, C₂- bis C₂₂-Alkenyl, C₆- bis C₃₀-Aryl- oder C₇-bis C₃₀-Alkylaryl steht, als Gegenionen für die Verbindungen gemäß Formel (1) alle Anionen geeignet sind, die die Löslichkeit der Verbindungen der Formel (1) in den organisch-wässrigen Mischphasen nicht beeinträchtigen, und wobei es sich bei den durch (O-A)ₘ bezeichneten Alkoxygruppen auch um gemischte Alkoxygruppen handeln kann.

Weiterhin wird ein Verfahren zur Inhibierung von Korrosion an Metalloberflächen, insbesondere von eisenhaltigen Metallen, indem einem korrosiven System, welches mit den Metalloberflächen in Kontakt steht, mindestens eine Verbindung der Formel (1) zugesetzt wird, beschrieben.

Weiterhin wird ein Verfahren zur Inhibierung von Gashydraten, indem einem zur Bildung von Gashydraten neigenden System aus Wasser und Kohlenwasserstoffen mindestens eine Verbindung der Formel (1) zugesetzt wird, beschrieben.

Ein weiterer Gegenstand der Erfindung ist die Verwendung von Verbindungen der 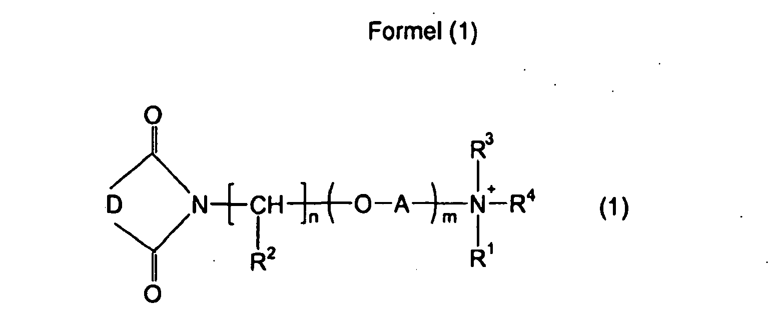 worin
- R¹: C₁- bis C₂₂-Alkyl, C₂- bis C₂₂-Alkenyl, C₆- bis C₃₀-Aryl- oder C₇- bis C₃₀-Alkylaryl, -CHR⁵-COO⁻ oder -O⁻,
- R²: Wasserstoff, -CH₃ oder -OH,
- R³, R⁴: unabhängig voneinander C₁- bis C₂₂-Alkyl, C₂- bis C₂₂-Alkenyl, C₆- bis C₃₀-Aryl- oder C₇- bis C₃₀-Alkylaryl,
- R⁵: Wasserstoff, C₁- bis C₂₂-Alkyl oder C₂- bis C₂₂-Alkenyl,
- A: eine C₂- bis C₄-Alkylengruppe,
- D: eine C₂- bis C₅-Alkylengruppe, die ein oder zwei Heteroatome enthalten und an jeder Position einen Substituenten R⁶ tragen kann,
- R⁶: einen beliebigen organischen Rest, der 1 bis 300 C-Atome enthält, und der Heteroatome enthalten kann,
- m: eine Zahl von 0 bis 30,
- n: eine Zahl von 1 bis 18 bedeuten,
wobei, wenn R¹ für C₁- bis C₂₂-Alkyl, C₂- bis C₂₂-Alkenyl, C₆- bis C₃₀-Aryl- oder C₇- bis C₃₀-Alkylaryl steht, als Gegenionen für die Verbindungen gemäß Formel (1) alle Anionen geeignet sind, die die Löslichkeit der Verbindungen der Formel (1) in den organisch-wässrigen Mischphasen nicht beeinträchtigen, und wobei es sich bei den durch (O-A)ₘ bezeichneten Alkoxygruppen auch um gemischte Alkoxygruppen handeln kann,
in Mengen von 5 bis 5000 ppm als Korrosionsinhibitoren und Gashydratinhibitoren..

Korrosive Systeme im Sinne dieser Erfindung sind bevorzugt flüssig/flüssig- bzw. flüssig/gasförmig-Mehrphasensysteme, enthaltend Wasser und Kohlenwasserstoffe, die in freier und/oder gelöster Form korrosive Bestandteile, wie Salze und Säuren, enthalten. Die korrosiven Bestandteile können auch gasförmig sein, wie etwa Schwefelwasserstoff und Kohlendioxid.

Kohlenwasserstoffe im Sinne dieser Erfindung sind organische Verbindungen, die Bestandteile des Erdöls/Erdgases sind, und deren Folgeprodukte. Kohlenwasserstoffe im Sinne dieser Erfindung sind auch leichtflüchtige Kohlenwasserstoffe, wie beispielsweise Methan, Ethan, Propan, Butan. Für die Zwecke dieser Erfindung zählen dazu auch die weiteren gasförmigen Bestandteile des Erdöls/Erdgases, wie etwa Schwefelwasserstoff und Kohlendioxid.

R¹, R³ und R⁴ stehen vorzugsweise unabhängig voneinander für eine Alkyl- oder Alkenylgruppe von 1 bis 14 Kohlenstoffatomen, insbesondere für solche Gruppen mit 1 bis 6 Kohlenstoffatomen und speziell für Methyl- oder Butyl-Gruppen.

Steht R¹ für C₁- bis C₂₂-Alkyl, C₂- bis C₂₂-Alkenyl, C₆- bis C₃₀-Aryl- oder C₇- bis C₃₀-Alkylaryl, so sind als Gegenionen für die Verbindungen gemäß Formel (1) alle Anionen geeignet, die die Löslichkeit der Verbindungen der Formel (1) in den organisch-wässrigen Mischphasen nicht beeinträchtigen. Solche Gegenionen sind beispielsweise Methylsulfationen (Methosulfat) oder Halogenidionen.

Steht R¹ für Reste -CHR⁵-COO⁻ bzw. -O⁻, so sind die Verbindungen der Formel (1) Betaine bzw. Aminoxide und besitzen als innere Salze (Ampholyte) ein intramolekulares Gegenion.

R² und R⁵ stehen vorzugsweise für Wasserstoff.

m steht vorzugsweise für eine Zahl zwischen 1 und 30, insbesondere zwischen 2 und 12, speziell 3 und 6.

n steht vorzugsweise für eine Zahl zwischen 2 und 12, insbesondere für 3 bis 6.

A kann geradkettig oder verzweigt sein und steht vorzugsweise für eine Ethylenoder Propylengruppe, insbesondere eine Ethylengruppe.

D erzeugt einen Ringschluss zwischen den Carbonylgruppen gemäß Formel 1. Die Ringgröße beträgt unter Einschluss der Carbonyl-Kohlenstoffatome und des Stickstoffatoms zwischen 5 und 8 Ringatomen. D ist somit eine Alkylengruppe mit 2 bis 5 Kohlenstoffatomen, die ein oder zwei Heteroatome enthalten kann.

D kann an jeder Position einen Substituenten R⁶ tragen.

R⁶ kann ein beliebiger organischer Rest sein, der 1 bis 300 C-Atome enthält, und der Heteroatome enthalten kann. Enthält R⁶ keine Heteroatome, so handelt es sich vorzugsweise um C₁- bis C₁₀₀-Alkyl- oder C₂- bis C₁₀₀-Alkenyl-Reste, die als Oligomere von C₂- bis C₈-Alkylenbausteinen abgeleitet sind, insbesondere von Ethylen, Propylen und Butylen.

Steht R⁶ für Alkyl- oder Alkenyl-Reste, können diese geradkettig oder verzweigt sein, vorzugsweise verzweigt. In einer besonderen Ausführungsform sind die verzweigten Alkyl- oder Alkenyl-Reste Polypropylen oder Polyisobutylen mit mehr als 12 Kohlenstoffatomen.

Enthält R⁶ Heteroatome, so handelt es sich vorzugsweise um Stickstoff- oder Sauerstoffatome oder beides, vorzugsweise um beides. Die Stickstoffatome können in quaternierter Form vorliegen.

R⁶ steht vorzugsweise für einen Rest der Formel (2) 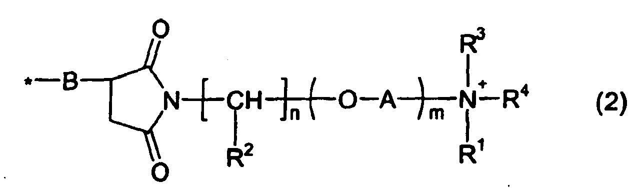 worin
B für einen C₁- bis C₁₀₀-Alkylen- oder C₂- bis C₁₀₀-Alkenylen-Rest steht, der als Oligomer von C₂- bis C₈-Alkylenbausteinen, insbesondere von Ethylen, Propylen und Butylen, abgeleitet ist und die Bindung an D in Formel (1) über eine freie Valenz einer Alkyl-Gruppe an beliebiger Stelle von B erfolgt.

R¹, R², R³, R⁴, m und n haben die bereits oben angegebenen Bedeutungen mit den jeweils weiter oben für R¹, R², R³, R⁴ , m und n angegebenen Vorzugsbereichen.

Die erfindungsgemäßen Verbindungen können alleine oder in Kombination mit anderen bekannten Korrosionsinhibitoren und/oder Gashydratinhibitoren eingesetzt werden. Im allgemeinen wird man soviel des erfindungsgemäßen Korrosionsinhibitors und/oder Gashydratinhibitoren einsetzen, dass man unter den gegebenen Bedingungen einen ausreichenden Korrosionsschutz und Schutz vor Gashydratbildung erhält.

Bevorzugte Einsatzkonzentrationen der Korrosionsinhibitoren bezogen auf die reinen erfindungsgemäßen Verbindungen sind 10 bis 1000, insbesondere 15 bis 150 ppm.

Die Gashydratinhibitoren werden im allgemeinen in Mengen zwischen 0,01 und 5 Gew.-% der reinen erfindungsgemäßen Verbindungen bezogen auf die wässrige Phase, vorzugsweise zwischen 0,05 und 2 Gew.-% verwendet.

Besonders geeignet als Korrosionsinhibitoren und/oder Gashydratinhibitoren sind auch Mischungen der erfindungsgemäßen Produkte mit anderen literaturbekannten Korrosionsinhibitoren und/oder Gashydratinhibitoren.

Besonders geeignet als Korrosionsinhibitoren und somit eine bevorzugte Ausführungsform dieser Erfindung sind Mischungen der Verbindungen gemäß Formel (1), wie mit Amidoaminen und/oder Imidazolinen aus Fettsäuren und Polyaminen und deren Salzen, quartären Ammoniumsalzen, Alkylpyridinen oxethylierten/oxpropylierten Aminen, Amphoglycinaten und -propionaten, Betainen oder Verbindungen beschrieben in DE-A-199 30 683.

Besonders geeignet als Gashydratinhibitoren und somit eine bevorzugte Ausführungsform dieser Erfindung sind Mischungen der Verbindungen gemäß Formel (1) mit einem oder mehreren Polymeren mit einem durch Polymerisation erhaltenen Kohlenstoff-Backbone und Amidbindungen in den Seitenketten. Hierzu zählen besonders Homopolymere und/oder Copolymere des Vinylpyrrolidons, Vinylcaprolactams, iso-Propylacrylamids, Acryloylpyrrolidins, N-Methyl-N-Vinylacetamid sowie weiterer anionischer, kationischer und neutraler Comonomere mit vinylischer Doppelbindung.

Werden Mischungen verwendet, so betragen die Konzentrationsverhältnisse zwischen den erfindungsgemäßen Gashydratinhibitoren und den zugemischten Komponenten von 90:10 bis 10:90 Gewichtsprozente, vorzugsweise werden Mischungen in den Verhältnissen 75:25 bis 25:75, und insbesondere von 60:40 bis 40:60 verwendet.

Die erfindungsgemäßen Verbindungen können dadurch hergestellt werden, dass Dicarbonsäurederivate, w.z.B. Alkenylbernsteinsäureanhydride, mit tertiären Alkylamino-alkyl/alkoxy-aminen zu den entsprechenden Dicarbonsäureimiden kondensiert werden. Anschließend wird mit geeigneten Alkylierungsmitteln quaterniert.

Die Herstellung von Alkenylbernsteinsäureanhydriden durch thermische oder katalysierte "En"-Reaktion ist im Stand der Technik beschrieben. Dabei werden Olefine, bevorzugt Olefine mit terminaler Doppelbindung, mit Maleinsäureanhydrid unter erhöhten Temperaturen umgesetzt. In Abhängigkeit von der Reaktionsführung, von der Art des verwendeten Olefins und vom verwendeten Molverhältnis werden Mono- und/oder Bisaddukte, gegebenenfalls Polyaddukte erhalten.

Die Herstellung der Alkylamino-alkyl/alkoxy-amine, wie Alkylendiamine oder auch Alkylaminoalkyletheramine ist im Stand der Technik beschrieben.

Basis der verwendeten tertiären Alkylamino-alkyl/alkoxy-amine sind vorzugsweise Alkylendiamine mit C₁- bis C₂₂-Alkylresten oder C₂- bis C₂₂-Alkenylresten, bevorzugt C₁- bis C₈-Dialkylamino-alkylenamine. Besonders geeignete Dialkylaminoalkylenamine sind beispielsweise N,N-Dibutylaminopropylamin, N,N-Diethylaminopropylamin, N,N-Dimethylamino-propylamin, N,N-Dimethylaminobutylamin, N,N-Dimethylaminohexylamin, N,N-Dimethylaminodecylamin, N,N-Dibutylaminoethylamin und N,N-Dimethylamino-2-hydroxypropylamin.

Die Alkenylbernsteinsäureanhydride werden im allgemeinen mit den Alkylendiaminen derart umgesetzt, dass unter Eliminierung von Reaktionswasser eine vollständige Kondensation zum Alkenylsuccinimid erfolgt. Der Umsetzungsgrad kann durch die Bestimmung der Säurezahl und/oder durch die Bestimmung des Basenstickstoffs verfolgt werden. Die Reaktion erfolgt bei 60 - 200°C, bevorzugt bei 120 - 160°C, um einen möglichst vollständigen Umsatz zu gewährleisten. Die verfahrensbedingte Bildung von entsprechenden Amiden als zwangsanfallende Nebenprodukte und die daraus resultierenden Folgeprodukte sind umfasst.

Die Umsetzung erfolgt in Substanz, kann aber auch bevorzugt in Lösung durchgeführt werden. Insbesondere wenn hohe Umsätze und niedrigere Säurezahlen von den resultierenden Alkenylsuccinimiden angestrebt werden, ist die Verwendung von Lösemitteln erforderlich. Geeignete Lösemittel für die Herstellung sind organische Verbindungen, durch die das Reaktionswasser azeotrop entfernt wird. Insbesondere können aromatische Lösemittel oder Lösemittelgemische, oder Alkohole verwendet werden. Besonders bevorzugt ist 2-Ethylhexanol. Die Reaktionsführung erfolgt dann beim Siedepunkt des Azeotrops.

Für die Herstellung der erfindungsgemäßen Quats werden in einem anschließenden Reaktionsschritt die Alkenylsuccinimidoalkylamine quaterniert. Die Quaternierung kann durch entsprechende Alkylierungsmittel bei 50 bis 150 °C erfolgen. Geeignete Alkylierungsmittel stellen Alkylhalogenide und Alkylsulfate dar, bevorzugt Methylchlorid, Methyljodid, Butylbromid und Dimethylsulfat.

Für die Herstellung der erfindungsgemäßen Betaine werden in einem anschließenden Reaktionsschritt die Alkenylsuccinimidoalkylamine mit einer Halogencarbonsäure und einer Base, bevorzugt Chloressigsäure und Natriumhydroxid umgesetzt. Dies kann geschehen, indem man die Alkenylsuccinimidoalkylamine mit 50 bis 125 Mol-% Halogencarbonsäure bei 40°C vorlegt und bei 40 bis 100°C durch einmalige oder portionierte Zugabe der Base und der zu 125 Mol-% verbleibenden Restmenge Halogencarbonsäure umsetzt.

Als basische Verbindungen können Erdalkali-/Alkalimetallhydroxide oder -alkoholate (Natriummethylat, Natriumethylat, Kalium-tert.-butylat) verwendet werden, bevorzugt sind aber Alkalimetallhydroxide, besonders Natriumhydroxid oder Kaliumhydroxid, insbesondere deren wässrigen Lösungen.

Die Herstellung der erfindungsgemäßen Aminoxide erfolgt nach bekannten Verfahren des Standes der Technik, vorzugsweise durch Oxidation der entsprechenden tertiären Amingruppe mit Peroxiden oder Persäuren, bevorzugt mit Wasserstoffperoxid.

Die Umsetzung zu den erfindungsgemäßen Verbindungen erfolgt bevorzugt in Lösung, kann aber auch in Substanz durchgeführt werden. Geeignete Lösemittel für die Herstellung der Quats, Betaine bzw. Aminoxide sind inerte Alkohole wie Isopropanol oder inerte Ether wie Tetrahydrofuran, Glyme, Diglyme und MPEGs.

In Abhängigkeit von den gegebenen Anforderungen kann das verwendete Lösemittel im erfindungsgemäßen Produkt verbleiben oder muss destillativ entfernt werden.

### Beispiele:

### a) Allgemeine Vorschrift für die Herstellung der Alkenylsuccinimido-alkylamine

In einer Rührapparatur mit Destillationsbrücke wurden 2,5 mol des entsprechenden Alkenylbernsteinsäureanhydrids (gemäß Verseifungszahl) unter Stickstoffspülung vorgelegt und auf 60°C erwärmt. Dann wurden 2,5 mol des entsprechenden Alkylendiamins über 2 Stunde zugetropft, wobei sich die Reaktionsmischung auf ca. 100°C erwärmte. Die Reaktionsmischung wurde 1 h bei 100°C nachgerührt und dann die Reaktionstemperatur über eine Periode von 8 Stunden kontinuierlich von 100°C auf 160°C gesteigert, wobei Reaktionswasser abdestillierte. Abschließend wurde 4 Stunden bei 160°C nachreagiert.

### Beispiel 1 (Dodecenyl-/Tetradecenylsuccinimido-N,N-dimethyl-propylamin)

Aus 671 g Dodecenyl-/Tetradecenyl-bernsteinsäureanhydrid (VZ = 418,1 mg KOH/g) und 255,5 g Dimethylaminopropylamin (DMAPA) wurden 875 g Dodecenyl-/Tetradecenylsuccinimido-N,N-dimethyl-propylamin mit SZ = 3,2 mg KOH/g und bas.-N = 4,03 % erhalten.

### Beispiel 2 (Tetrapropylensuccinimido-N,N-dimethyl-propylamin)

Aus 732,0 g Tetrapropylen-bernsteinsäureanhydrid (VZ = 383,3 mg KOH/g) und 255,5 g Dimethylaminopropylamin (DMAPA) wurden 945 g Tetrapropylensuccinimido-N,N-dimethyl-propylamin mit SZ = 10,3 mg KOH/g und bas.-N = 3,73 % erhalten.

### Beispiel 3 (Pentapropylensuccinimido-N,N-dimethyl-propylamin)

Aus 978,5 g Pentapropylen-bernsteinsäureanhydrid (VZ = 286,7 mg KOH/g) und 255,5 g Dimethylaminopropylamin (DMAPA) wurden 1181 g Pentapropylensuccinimido-N,N-dimethyl-propylamin mit SZ = 15,1 mg KOH/g und bas.-N = 2,87 % erhalten.

### Beispiel 4 (Polyisobutenylsuccinimido-N,N-dimethyl-propylamin)

Aus 978,3 g Polyisobutenyl-bernsteinsäureanhydrid (auf Basis PIB 300; VZ = 286,8 mg KOH/g) und 255,5 g Dimethylaminopropylamin (DMAPA) wurden 1180 g Polyisobutenylsuccinimido-N,N-dimethyl-propylamin mit SZ = 9,7 mg KOH/g und bas.-N = 2,96 % erhalten.

### Beispiel 5 (Polyisobutenylsuccinimido-N,N-dirnethyl-propylamin)

Aus 1310 g (2 mol) Polyisobutenyl-bernsteinsäureanhydrid (auf Basis PIB 550; VZ = 171,3 mg KOH/g) und 204,0 g (2 mol) Dimethylaminopropylamin (DMAPA) wurden 1468 g Polyisobutenylsuccinimido-N,N-dimethyl-propylamin mit SZ = 6,7 mg KOH/g und bas.-N = 1,89 % erhalten.

### Beispiel 6 (Polyisobuteny)-succinimido-N,N-dimethy)-propylamin)

Aus 870,3 g (2 mol) Polyisobutenyl-bernsteinsäureanhydrid (auf Basis PIB 550; VZ = 257,9 mg KOH/g) und 204,0 g (2 mol) Dimethylaminopropylamin (DMAPA) wurden 1468 g Polyisobutenylsuccinimido-N,N-dimethyl-propylamin mit SZ = 15,4 mg KOH/g und bas.-N = 2,69 % erhalten.

### b) Allgemeine Vorschrift für die Herstellung der Succinimidoammonium-betaine

In einer Rührapparatur wurden 2 mol (gemäß bas.-N) des entsprechenden Alkenylsuccinimido-alkylamins unter Stickstoffspülung vorgelegt und in 40 Gew.-% Isopropanol (bezogen auf die Gesamtmenge) bei 40°C unter kontinuierlichem Rühren gelöst. Dann wurde 2,5 mol Monochloressigsäure in einer Portion zugegeben und homogen verrührt. Anschließend wurden zu dieser Reaktionsmischung in 4 Portionen 2,7 mol einer wässrigen NaOH-Lösung (216 g einer 50 %igen Lösung) zulaufen lassen. Die Zugabe erfolgte so, dass die Innentemperatur 60°C nicht überstieg (ggf. war Kühlung erforderlich). Nach jeder Zugabe wurde jeweils 30 Minuten, zum Abschluß 4 Stunden bei 80°C nachreagiert. Abschließend wurde der ausgefallene NaCl Rückstand über Seitz T 5500 mittels einer Druckfilterpresse abgetrennt.

### Beispiel 7 (Dodecenyl-/Tetradecenylsuccinimido-N,N-dimethyl-propylammonium-N-methylcarboxy-betain)

Aus 695,3 g Dodecenyl-/Tetradecenylsuccinimido-N,N-dimethyl-propylamin (bas.-N = 4,03 %) und 236,3 g Monochloressigsäure (MCAA) wurden 1600 g Dodecenyl-/Tetradecenylsuccinimido-N,N-dimethyl-propylammonium-N-methylcarboxy-betain (WS-Gehalt ca. 54 %, ca. 6 % Wasser, ca. 40 % Isopropanol) erhalten.

### Beispiel 8 (Tetrapropylensuccinimido-N,N-dimethyl-propylammonium-N-methylcarboxy-betain)

Aus 751,2 g Tetrapropylensuccinimido-N,N-dimethyl-propylamin (bas.-N = 3,73 %) und 236,3 g Monochloressigsäure (MCAA) wurden 1710 g Tetrapropylensuccinimido-N,N-dimethyl-propylammonium-N-methylcarboxy-betain (WS-Gehalt ca. 54 %, ca. 6 % Wasser, ca. 40 % Isopropanol) erhalten.

### Beispiel 9 (Pentapropylensuccinimido-N,N-dimethyl-propylammonium-N-methylcarboxy-betain)

Aus 976,3 g Pentapropylensuccinimido-N,N-dimethyl-propylamin (bas.-N = 2,87 %) und 236,3 g Monochloressigsäure (MCAA) wurden 2080 g Pentapropylensuccinimido-N,N-dimethyl-propylammonium-N-methylcarboxy-betain (WS-Gehalt ca. 54 %, ca. 6 % Wasser, ca. 40 % Isopropanol) erhalten.

### Beispiel 10 (Polyisobutenylsuccinimido-N,N-dimethyl-propylammonium-N-methylcarboxy-betain)

Aus 946,0 g Polyisobutenylsuccinimido-N,N-dimethyl-propylamin (auf Basis PIB 300; bas.-N = 2,96 %) 236,3 g Monochloressigsäure (MCAA) wurden 2035 g Polyisobutenylsuccinimido-N,N-dimethyl-propylammonium-N-methylcarboxy-betain (WS-Gehalt ca. 54 %, ca. 6 % Wasser, ca. 40 % Isopropanol) erhalten.

### Beispiel 11 (Polyisobutenylsuccinimido-N,N-dimethyl-propylammonium-N-methylcarboxy-betain)

Aus 741 g (1 mol) Polyisobutenylsuccinimido-N,N-dimethyl-propylamin (auf Basis PIB 550; bas.-N = 1,89 %) und 118,2 g (1,25 mol Monochloressigsäure (MCAA) wurden 1415 g Polyisobutenylsuccinimido-N,N-dimethyl-propylammonium-N-methylcarboxy-betain (WS-Gehalt ca. 54 %, ca. 6 % Wasser, ca. 40 % Isopropanol) erhalten.

### Beispiel 12 (Polyisobutenyl-succinimido-N,N-dimethyl-propylammonium-N-methylcarboxy-betain)

Aus 520,8 g (1 mol)) Polyisobutenylsuccinimido-N,N-dimethyl-propylamin (auf Basis PIB 550; bas.-N = 2,69 %) und 118,2 g (1,25 mol Monochloressigsäure (MCAA) wurden 1090 g Polyisobutenylsuccinimido-N,N-dimethyl-propylammonium-N-methylcarboxy-betain (WS-Gehalt ca. 54 %, ca. 6 % Wasser, ca. 40 % Isopropanol) erhalten.

### Wirksamkeit der erfindungsgemäßen Verbindungen als Korrosionsinhibitoren

Die erfindungsgemäßen Verbindungen wurden als Korrosionsinhibitoren im Shell-Wheel-Test geprüft. Coupons aus C-Stahl (DIN 1.1203 mit 15 cm² Oberfläche) wurden in eine Salzwasser/Petroleum-Mischung (9:1,5 %ige NaCl-Lösung mit Essigsäure auf pH 3,5 gestellt) eingetaucht und bei einer Umlaufgeschwindigkeit von 40 rpm bei 70°C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

In den folgenden Tabellen bezeichnet "Vergleich" ein handelsübliches Sojafettsäureamidopropyl-N,N-dimethylammonium-carboxymethyl-betain beschrieben durch EP-B-0 446 616 (Korrosionsinhibitor des Standes der Technik).

**Tabelle 1:**

| (SHELL-Wheel-Test) | | |
|---|---|---|
| Beispiel | Korrosionsinhibitor tel quel | ø Schutz % |
| Vergleich | | 75,4 |
| 13 | Betain aus Beispiel 7 | 84,9 |
| 14 | Betain aus Beispiel 8 | 84,0 |
| 15 | Betain aus Beispiel 9 | 90,4 |
| 16 | Betain aus Beispiel 10 | 90,1 |
| 17 | Betain aus Beispiel 11 | 80,6 |
| 18 | Betain aus Beispiel 12 | 82,7 |

Die Produkte wurden außerdem im LPR-Test (Testbedingungen analog ASTM D 2776) geprüft.

**Tabelle 2:**

| (LPR- Test) | | | | |
|---|---|---|---|---|
| Beispiel | Korrosionsinhibitor | Schutz nach [%] | | |
| | | 10 min | 30 min | 60 min |
| Vergleich | | 45,9 | 59,2 | 64,3 |
| 19 | Betain aus Beispiel 7 | 72,5 | 92,0 | 98,1 |
| 20 | Betain aus Beispiel 8 | 74,0 | 81,3 | 84,9 |
| 21 | Betain aus Beispiel 9 | 77,9 | 94,0 | 99,8 |
| 22 | Betain aus Beispiel 10 | 79,1 | 92,3 | 99,2 |
| 23 | Betain aus Beispiel 11 | 61,9 | 70,6 | 80,6 |
| 24 | Betain aus Beispiel 12 | 70,0 | 79,4 | 86,3 |

Wie aus den obigen Testresultaten zu erkennen ist, weisen die erfindungsgemäßen Produkte sehr gute Korrosionsschutzeigenschaften bei niedriger Dosierung auf und übertreffen die Performance der Inhibitoren des Standes der Technik.

Die Schaumeigenschaften wurden mit der Schüttelschaum-Methode überprüft. Dazu wurden 50 ml einer 3 %igen wässrigen Lösung des entsprechenden Korrosionsinhibitors in VE-Wasser in einem verschlossenen 100 ml - Messzylinder innerhalb von 10 sec. 20ig mal geschüttelt. Für die Beurteilung des Schaumverhaltens wurden nach Beendigung des Schüttelns das Gesamtvolumen der Lösung (Schaumhöhe) und die Schaumzerfallzeit (Zeit bis Erreichen des Ausgangsvolumens von 50 ml) herangezogen. Im allgemeinen ist dieses Prüfverfahren mäßig reproduzierbar, eignet sich jedoch hervorragend für eine tendenzielle Abschätzung des Schaumverhaltens in schwach schäumend, schäumend oder stark schäumend.

**Tabelle 3:**

| (Schüttelschaum) | | |
|---|---|---|
| Beispiel | Korrosionsinhibitor | Schaumverhalten |
| Vergleich | | stark schäumend |
| 25 | Betain aus Beispiel 7 | schäumend |
| 26 | Betain aus Beispiel 8 | schäumend |
| 27 | Betain aus Beispiel 9 | schwach schäumend |
| 28 | Betain aus Beispiel 10 | schwach schäumend |
| 29 | Betain aus Beispiel 11 | schwach schäumend |
| 30 | Betain aus Beispiel 12 | schäumend |

Die Verbindungen sind biologisch abbaubar und besitzen ein besseres Schaumverhalten als die Korrosionsinhibitoren des Standes der Technik.

## Patentansprüche

1. Verwendung von Verbindungen der Formel (1) 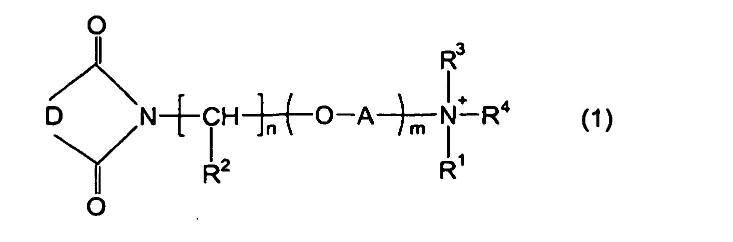 worin
R¹ C₁- bis C₂₂-Alkyl, C₂- bis C₂₂-Alkenyl, C₆- bis C₃₀-Aryl- oder C₇- bis C₃₀-Alkylaryl, -CHR⁵-COO⁻ oder -O⁻,
R² Wasserstoff, -CH₃ oder -OH,
R³, R⁴ unabhängig voneinander C₁- bis C₂₂-Alkyl, C₂- bis C₂₂-Alkenyl, C₆- bis C₃₀-Aryl- oder C₇- bis C₃₀-Alkylaryl,
R⁵ Wasserstoff, C₁- bis C₂₂-Alkyl oder C₂- bis C₂₂-Alkenyl,
A eine C₂- bis C₄-Alkylengruppe,
D eine C₂- bis C₅-Alkylengruppe, die ein oder zwei Heteroatome enthalten und an jeder Position einen Substituenten R⁶ tragen kann,
R⁶ einen beliebigen organischen Rest, der 1 bis 300 C-Atome enthält, und der Heteroatome enthalten kann,
m eine Zahl von 0 bis 30,
n eine Zahl von 1 bis 18 bedeuten,
wobei, wenn R¹ für C₁- bis C₂₂-Alkyl, C₂- bis C₂₂-Alkenyl, C₆- bis C₃₀-Aryl- oder C₇- bis C₃₀-Alkylaryl steht, als Gegenionen für die Verbindungen gemäß Formel (1) alle Anionen geeignet sind, die die Löslichkeit der Verbindungen der Formel (1) in den organisch-wässrigen Mischphasen nicht beeinträchtigen, und wobei es sich bei den durch (O-A)ₘ bezeichneter Alkoxygruppen auch um gemischte Alkoxygruppen handeln kann,
in Mengen von 5 bis 5000 ppm als Korrosionsinhibitoren und Gashydratinhibitoren.

2. Verwendung nach Anspruch 1, worin R³ und R⁴ für eine Alkyl- oder Alkenylgruppe von 1 bis 14 Kohlenstoffatomen stehen.

3. Verwendung nach Anspruch 1 und/oder 2, worin R² für Wasserstoff steht.

4. Verwendung nach einem oder mehreren der Ansprüche 1 bis 3, worin n für eine Zahl zwischen 2 und 12 steht.

5. Verwendung nach einem oder mehreren der Ansprüche 1 bis 4, worin m für eine Zahl zwischen 1 und 30 steht.

6. Verwendung nach einem oder mehreren der Ansprüche 1 bis 5, worin D einen Rest R⁶ umfasst, welcher ein C₁- bis C₁₀₀-Alkyl- oder C₂- bis C₁₀₀-Alkenyl-Rest ist, der ein Oligomer von C₂- bis C₈-Alkylenen ist.

7. Verwendung nach einem oder mehreren der Ansprüche 1 bis 5, worin D einen Rest R⁶ umfasst, welcher Stickstoff- oder Sauerstoffatome oder beides umfasst.

8. Verwendung nach einem oder mehreren der Ansprüche 1 bis 5, worin D einen Rest R⁶ umfasst, welcher ein Rest der Formel 2 ist 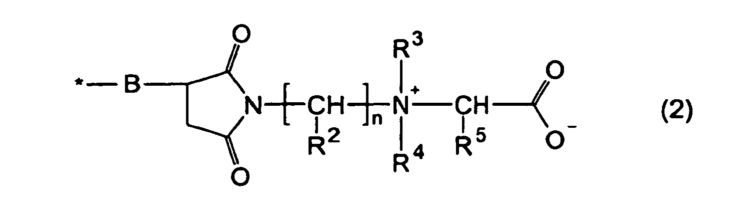 worin
B für einen C₁- bis C₁₀₀-Alkylen- oder C₂- bis C₁₀₀-Alkenylen-Rest steht, der als Oligomer von C₂- bis C₈-Alkylenbausteinen abgeleitet ist und die Bindung an D in Formel 1 über eine freie Valenz einer Alkylgruppe an beliebiger Stelle von B erfolgt.

9. Verbindungen der Formel (1) 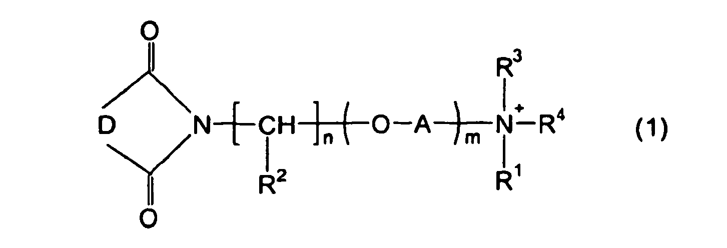 worin
R¹ C₁- bis C₂₂-Alkyl, C₂- bis C₂₂-Alkenyl, C₆- bis C₃₀-Aryl- oder C₇- bis C₃₀- Alkylaryl, -CHR⁵-COO⁻ oder -O⁻,
R² Wasserstoff, -CH₃ oder -OH,
R³, R⁴ unabhängig voneinander C₁- bis C₂₂-Alkyl, C₂- bis C₂₂-Alkenyl, C₆- bis C₃₀-Aryl- oder C₇- bis C₃₀-Alkylaryl,
R⁵ Wasserstoff, C₁- bis C₂₂-Alkyl oder C₂- bis C₂₂-Alkenyl,
A eine C₂- bis C₄-Alkylengruppe,
D eine C₂-Alkylengruppe, die an jeder Position einen Substituenten R⁶ tragen kann,
R⁶ einen beliebigen organischen Rest, der 1 bis 300 C-Atome enthält, und der Heteroatome enthalten kann,
m eine Zahl von 1 bis 30,
n eine Zahl von 1 bis 18 bedeuten,
wobei, wenn R¹ für C₁- bis C₂₂-Alkyl, C₂- bis C₂₂-Alkenyl, C₆- bis C₃₀-Aryl- oder C₇- bis C₃₀-Alkylaryl steht, als Gegenionen für die Verbindungen gemäß Formel (1) alle Anionen geeignet sind, die die Löslichkeit der Verbindungen der Formel (1) in den organisch-wässrigen Mischphasen nicht beeinträchtigen, und wobei es sich bei den durch (O-A)ₘ bezeichneten Alkoxygruppen auch um gemischte Alkoxygruppen handeln kann.

## Claims

1. The use of compounds of the formula (1) 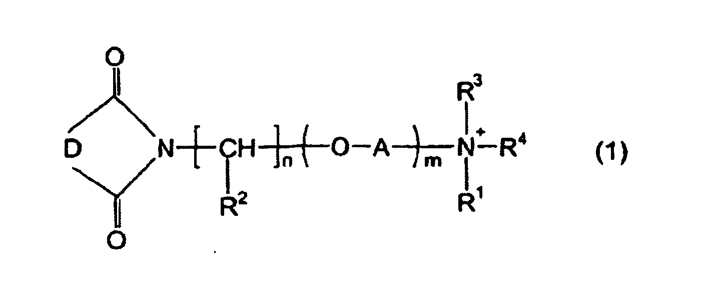 where
R¹ is C₁- to C₂₂-alkyl, C₂- to C₂₂-alkenyl, C₆- to C₃₀-aryl or C₇- to C₃₀-alkylaryl, -CHR⁵-COO⁻ or -O⁻,
R² is hydrogen -CH₃ or -OH,
R³, R⁴ are each independently C₁- to C₂₂-alkyl, C₂- to C₂₂-alkenyl, C₆- to C₃₀-aryl or C₇- to C₃₀-alkylaryl,
R⁵ is hydrogen, C₁- to C₂₂-alkyl or C₂- to C₂₂-alkenyl,
A is a C₂- to C₄-alkylene group,
D is a C₂- to C₅-alkylene group which may contain one or two heteroatoms and may bear an R⁶ substituent at any position,
R⁶ is any desired organic radical which contains from 1 to 300 carbon atoms and which may contain heteroatoms,
m is a number from 0 to 30,
n is a number from 1 to 18,
where, when R¹ is C₁- to C₂₂-alkyl, C₂- to C₂₂-alkenyl, C₆- to C₃₀-aryl or C₇- to C₃₀- alkylaryl, suitable counterions for the compounds of the formula (1) are all anions which do not impair the solubility of the compounds of the formula (1) in the organic-aqueous mixed phases, and where the alkoxy groups denoted by (O-A)ₘ may also be mixed alkoxy groups in amounts of from 5 to 5000 ppm as corrosion inhibitors and gas hydrate inhibitors.

2. The use as claimed in claim 1, wherein R³ and R⁴ are each an alkyl or alkenyl group having from 1 to 14 carbon atoms.

3. The use as claimed in claim 1 and/or 2, wherein R² is hydrogen.

4. The use as claimed in one or more of claims 1 to 3, wherein n is a number in the range from 2 to 12.

5. The use as claimed in one or more of claims 1 to 4, wherein m is a number in the range from 1 to 30.

6. The use as claimed in one or more of claims 1 to 5, wherein D includes an R⁶ radical which is a C₁- to C₁₀₀-alkyl or C₂- to C₁₀₀-alkenyl radical which is an oligomer of C₂- to C₈-alkylenes.

7. The use as claimed in one or more of claims 1 to 5, wherein D includes an R⁶ radical which includes nitrogen or oxygen atoms or both.

8. The use as claimed in one or more of claims 1 to 5, wherein D includes an R⁶ radical which is a radical of the formula 2 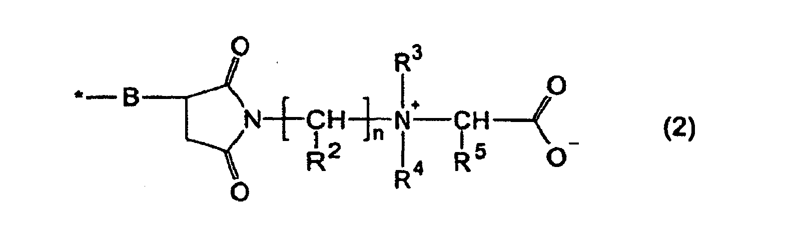 where
B is a C₁- to C₁₀₀-alkylene or C₂- to C₁₀₀-alkenylene radical which is an oligomer C₂- to C₈-alkylene building blocks and the bond to D in formula 1 is via a free valence of an alkyl group at any desired point on B.

9. A compound of the formula (1) 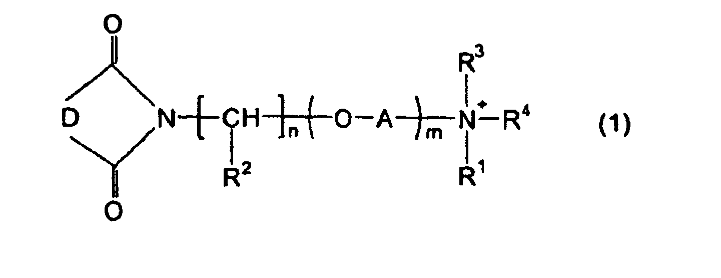 where
R¹ is C₁- to C₂₂-alkyl, C₂- to C₂₂-alkenyl, C₆- to C₃₀-aryl or C₇- to C₃₀-alkylaryl, -CHR⁵-COO⁻ or -O⁻,
R² is hydrogen -CH₃ or -OH,
R³, R⁴ are each independently C₁- to C₂₂-alkyl, C₂- to C₂₂-alkenyl, C₆- to C₃₀-aryl or C₇- to C₃₀-alkylaryl,
R⁵ is hydrogen, C₁- to C₂₂-alkyl or C₂- to C₂₂-alkenyl,
A is a C₂- to C₄-alkylene group,
D is a C₂-alkylene group which may bear an R⁶ substituent at any position,
R⁶ is any desired organic radical which contains from 1 to 300 carbon atoms and which may contain heteroatoms,
m is a number from 0 to 30,
n is a number from 1 to 18,
where, when R¹ is C₁- to C₂₂-alkyl, C₂- to C₂₂-alkenyl, C₆- to C₃₀-aryl or C₇- to C₃₀- alkylaryl, suitable counterions for the compounds of the formula (1) are all anions which do not impair the solubility of the compounds of the formula (1) in the organic-aqueous mixed phases, and where the alkoxy groups denoted by (O-A)ₘ may also be mixed alkoxy groups.

## Revendications

1. Utilisation de composés de formule (1) 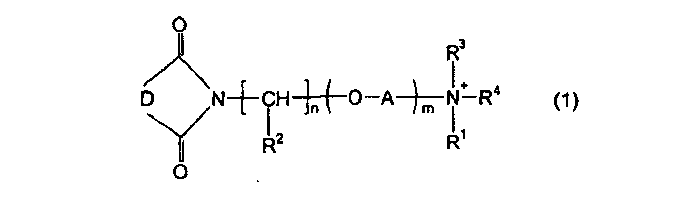 dans laquelle
R¹ représente un groupe alkyle en C₁-C₂₂, alcényle en C₂-C₂₂, aryle en C₆-C₃₀ ou alkylaryle en C₇-C₃₀, -CHR⁵-COO⁻ ou -O⁻,
R² représente un atome d'hydrogène, -CH₃ ou -OH,
R³, R⁴ représentent, indépendamment l'un de l'autre, un groupe alkyle en C₁-C₂₂, alcényle en C₂-C₂₂, aryle en C₆-C₃₀ ou alkylaryle en C₇-C₃₀,
R⁵ représente un atome d'hydrogène, un groupe alkyle en C₁-C₂₂ ou alcényle en C₂-C₂₂,
A représente un groupe alkylène en C₂-C₄,
D représente un groupe alkylène en C₂-C₅ qui peut contenir un ou deux hétéroatomes et porter en toute position un substituant R⁶,
R⁶ représente un radical organique quelconque qui contient de 1 à 300 atomes de carbone, et qui peut contenir des hétéroatomes,
m représente un nombre de 0 à 30,
n représente un nombre de 1 à 18,
lorsque R¹ représente un groupe alkyle en C₁-C₂₂, alcényle en C₂-C₂₂, aryle en C₆-C₃₀ ou alkylaryle en C₇-C₃₀, tous les anions qui n'altèrent pas la solubilité des composés de formule (1) dans les phases mixtes aqueuses-organiques étant appropriés en tant qu'ions opposés pour les composés selon la formule (1), et les groupes alcoxy désignés par (O-A)ₘ pouvant également consister en des groupes alcoxy mixtes, en quantités de 5 à 5 000 ppm en tant qu'agents anticorrosion et inhibiteurs de la formation d'hydrates de gaz.

2. Utilisation selon la revendication 1, dans laquelle R³ et R⁴ représentent un groupe alkyle ou alcényle ayant de 1 à 14 atomes de carbone.

3. Utilisation selon la revendication 1 et/ou la revendication 2, dans laquelle R² représente un atome d'hydrogène.

4. Utilisation selon une ou plusieurs des revendications 1 à 3, dans laquelle n représente un nombre compris entre 2 et 12.

5. Utilisation selon une ou plusieurs des revendications 1 à 4, dans laquelle m représente un nombre compris entre 1 et 30.

6. Utilisation selon une ou plusieurs des revendications 1 à 5, dans laquelle D comprend un radical R⁶ qui est un groupe alkyle en C₁-C₁₀₀ ou alcényle en C₂-C₁₀₀, qui est un oligomère d'alkylènes en C₂-C₈.

7. Utilisation selon une ou plusieurs des revendications 1 à 5, dans laquelle D comprend un radical R⁶ qui comprend des atomes d'oxygène ou d'azote.

8. Utilisation selon une ou plusieurs des revendications 1 à 5, dans laquelle D comprend un radical R⁶ qui est un radical de formule 2 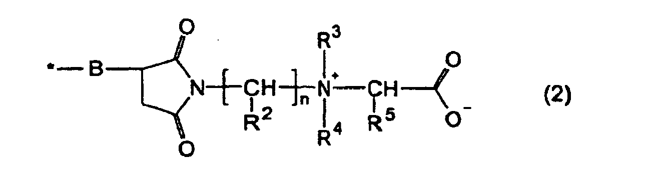 dans laquelle
B représente un radical alkylène en C₁-C₁₀₀ ou alcénylène en C₂-C₁₀₀, qui est dérivé en tant qu'oligomère de composants alkylène en C₂-C₈ et la liaison à D dans la formule 1 s'effectue par une valence libre d'un groupe alkyle en position quelconque de B.

9. Composés de formule (1) 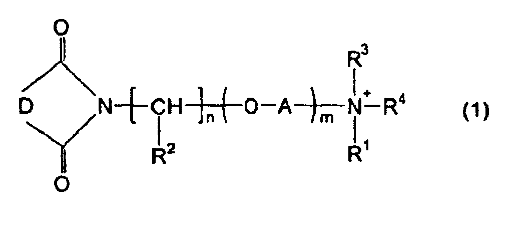 dans laquelle
R¹ représente un groupe alkyle en C₁-C₂₂, alcényle en C₂-C₂₂, aryle en C₆-C₃₀ ou alkylaryle en C₇-C₃₀, -CHR⁵-COO⁻ ou -O⁻,
R² représente un atome d'hydrogène, -CH₃ ou -OH,
R³, R⁴ représentent, indépendamment l'un de l'autre, un groupe alkyle en C₁-C₂₂, alcényle en C₂-C₂₂, aryle en C₆-C₃₀ ou alkylaryle en C₇-C₃₀,
R⁵ représente un atome d'hydrogène, un groupe alkyle en C₁-C₂₂ ou alcényle en C₂-C₂₂,
A représente un groupe alkylène en C₂-C₄,
D représente un groupe alkylène en C₂ qui peut porter en toute position un substituant R⁶,
R⁶ représente un radical organique quelconque qui contient de 1 à 300 atomes de carbone, et qui peut contenir des hétéroatomes,
m représente un nombre de 1 à 30,
n représente un nombre de 1 à 18,
lorsque R¹ représente un groupe alkyle en C₁-C₂₂, alcényle en C₂-C₂₂, aryle en C₆-C₃₀ ou alkylaryle en C₇-C₃₀, tous les anions qui n'altèrent pas la solubilité des composés de formule (1) dans les phases mixtes aqueuses-organiques étant appropriés en tant qu'ions opposés pour les composés selon la formule (1), et les groupes alcoxy désignés par (O-A)ₘ pouvant également consister en des groupes alcoxy mixtes.
